(19) Europäisches Patentamt
European Patent Office
Office européen des brevets

(11) **EP 1 319 417 A1**

(12) **EUROPEAN PATENT APPLICATION**

(43) Date of publication:
**18.06.2003 Bulletin 2003/25**

(51) Int Cl.[7]: **A61M 1/36**, A61M 5/168

(21) Application number: **01129940.1**

(22) Date of filing: **17.12.2001**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI**<br><br>(71) Applicant: **B. Braun Medizintechnologie GmbH**<br>**34212 Melsungen (DE)** | (72) Inventor: **Ferri, Angelo**<br>**41037 Mirandola (MO) (IT)**<br><br>(74) Representative: **Selting, Günther, Dipl.-Ing. et al**<br>**Patentanwälte**<br>**von Kreisler, Selting, Werner**<br>**Postfach 10 22 41**<br>**50462 Köln (DE)** |

(54) **Method and apparatus for monitoring a fluid conduit system of a medical instrument**

(57) The method for monitoring a fluid conduit system (12) connected to a medical instrument (10) comprises the following process steps: measuring the fluid pressure in the fluid conduit system (12), injecting or extracting a fluid test quantity into the fluid conduit system (12), measuring the fluid pressure in the fluid conduit system (12), determining the pressure difference between the initial and the final fluid pressures, comparing the determined pressure difference with a stored pressure-difference standard value, and issueing the deviation of the determined pressure difference from the stored pressure-difference standard value. The monitoring method allows the fluid conduit system to be monitored in a simple way with regard to certain characteristics, such as overall volume, gas volume contained, tightness etc.

Fig.1

EP 1 319 417 A1

## Description

The invention relates to methods for monitoring a fluid conduit system connected with a medical instrument, and to a medical instrument comprising a monitoring apparatus for monitoring a fluid conduit system.

A plurality of medical instruments is known which comprise fluid conduit systems and/or are connected with a patient's body via an extracorporeal fluid conduit system. Depending on the type of medical instrument used the fluid conduit system may be an internal conduit, a discharge conduit, a supply conduit or a combined discharge and supply conduit. To prevent unnecessary risks of infection, a new set of the fluid conduit system is used for the treatment of each patient. Such a set is also referred to as fluid conduit kit. Frequently used fluid conduit kits are, besides simple infusion conduit kits, in particular dialysis conduit kits.

A conduit kit normally comprises a plurality of sterilized plastic fluid conduits and other metal and plastic parts, such as filters, drip chambers, needles etc. The composition of a fluid conduit kit differs from therapy to therapy. Despite very careful in-process inspections it may happen that a conduit kit is defective, e. g. leaky, at the time of application. Further, it cannot be completely precluded that a conduit kit is installed which is unsuitable for the selected treatment. Such defects are frequently noticed only during the treatment or pass unnoticed.

It is an object of the invention to provide a method and an apparatus for monitoring a fluid conduit system for medical applications.

This object is solved with the features of claims 1, 2, 6 and 7, respectively.

According to method claim 1, after a first measurement of the initial fluid pressure, a fluid test quantity is injected or extracted by a fluid pump into or out of the fluid conduit system connected to the medical instrument. Then the final fluid pressure in the fluid conduit is measured. The difference between the two measured fluid pressure values is compared with a stored fluid pressure-difference standard value. Finally the deviation of the measured fluid pressure difference from the stored fluid pressure-difference standard value is issued.

The fluid pressure measured after injection or extraction of a fluid test quantity into or out of the fluid conduit system, i.e. a changement of the fluid quantity in the amount of the test fluid quantity, and the fluid pressure difference depend on a plurality of factors, inter alia, on the type of the connected fluid conduit kit or system, leakages in the fluid conduit, the liquid volume and/or the gas volume in the fluid conduit system or their ratio to each other. The stored fluid pressure-difference standard value is the fluid pressure difference to be expected after injection/extraction of the fluid test quantity provided that the fluid conduit kit involved has been correctly selected, does not show any undesired leakages and is filled with the correct quantities of liquid and gas. In the case of only a negligible deviation of the fluid pressure difference from the stored fluid pressure-difference standard value after injection/extraction of the fluid test quantity, it may be proceeded from the assumption that the fluid conduit system or kit connected to the medical instrument has been correctly selected, correctly connected, does not show any undesired leakages and/or contains the correct liquid and gas volumes.

Monitoring of the fluid conduit is effected as soon as the fluid conduit kit is connected to the medical instrument and prepared for treatment purposes, but prior to commencement of the treatment. In most treatment processes there are provided in or on the medical instrument a fluid pump as well as a pressure sensor for controlling the treatment and designed to be connected to the fluid conduit. In this case the elements, namely fluid pump and pressure sensor, already provided on the medical instrument can be used for monitoring purposes. The monitoring method is also suitable for controlling the fluid conduit during or after production.

In this way, a very simple method is provided for testing a fluid conduit or an extracorporal fluid conduit kit or system, which is connected to the medical instrument, immediately before commencement of the treatment, which testing relates to both the type of fluid conduit system connected and its tightness and liquid or gas filling. It is also possible to control fluid conduits during or right after their production. In the case of a larger deviation of the fluid pressure difference, determined after injection/extraction of the fluid test quantity, from the stored fluid pressure-difference standard value e. g. an alarm signal can be issued and taking into operation of the instrument may be prevented.

According to independent method claim 2, the fluid is a liquid and a gas, wherein the fluid pressure can be measured as the gas pressure in a gas bubble in the fluid conduit system. Gases are compressible such that by provision of a small gas bubble in the fluid conduit system overstretching of and possibly damage to the fluid conduit by injection of a liquid fluid test quantity are prevented.

Such a small gas bubble is provided anyway in most fluid conduit system kits, namely in the form of a gas-filled conduit branching off the main fluid conduit, wherein a pressure sensor is connected to the branch conduit, often behind a hydrophobic membrane. The hydrophobic membrane protects the pressure sensor, which is to be reused, against contamination by the liquid in the fluid conduit and is also referred to as gas pressure sensor protector. For the purpose of protecting the pressure sensor the hydrophobic membrane seals against the passage of liquid and gas as soon as it is wetted with liquid. The wetting has the further effect that the pressure sensor senses pressure changes to a considerably smaller degree such that the pressure signals are incorrect and may lead to incorrect conclusions during the treatment.

The monitoring method can be applied at regular or irregular intervals during the treatment such that from the measured fluid pressures and fluid pressure differences conclusions may be drawn with respect to the size of the gas bubble in the fluid conduit or in the area of the pressure sensor and the hydrophobic membrane. In this way it is, in turn, possible to determine whether wetting of the hydrophobic membrane is imminent or has already taken place.

It is generally possible to extract a corresponding fluid test quantity instead of or in addition to injection of a fluid test quantity into the fluid conduit system. It is also possible to extract and inject periodically and with a frequency lower than 10 Hz and to check the reaction continuously.

With the elasticity of the fluid conduit system and the low heat loss at small temperature changes being neglected, the gas bubble experiences an adiabatic change of condition due to injection/extraction of a defined fluid test quantity. Thus the following applies:

$$P \, V = n \, R \, T,$$

where

P is the gas pressure,
V is the gas volume,
n is the gas quantity in mol,
R is the Boltzmann's constant (8.3143 Joule/(mol Kelvin)), and
T is the absolute gas temperature in Kelvin.

For small volume changes the temperature T is assumed to be constant such that the term $n \, R \, T$ is constant provided that no gas escapes from or invades into the volume. Thus the following applies:

$$P \, V = constant$$

This means that a small change in the gas volume effects a corresponding inversely proportional pressure change in the gas volume.

This shows that, in the case of a known volume change caused by injection/extraction of the fluid test quantity, the gas pressure in the gas bubble must change inversely proportionally. If this is not the case, this indicates a leakage in the fluid conduit system. Proceeding from the assumption that the fluid conduit system is gastight, the gas volume of the gas bubble can be determined by measuring the gas pressure alone:

Since

$$P_1 \, V_0 = P_2 \, (V_0 + \Delta V) = n \, R \, T$$

is considered to be constant for small gas temperature changes, the gas volume is calculated from

$$V_0 = -\Delta V \, P_2 \, / \, (P_2 - P_1), \qquad (1)$$

where

$V_0$ is the initial gas volume,
$\Delta V$ is the known gas volume change corresponding to the fluid test quantity,
$P_1$ is the initial gas pressure prior to injection/extraction of the fluid test quantity, and
$P_2$ is the final gas pressure after injection/extraction of the fluid test quantity.

In this way it can e. g. be established whether the gas volume in the area of the gas pressure sensor protector is so large that the membrane is not wetted and the gas pressure sensor can supply correct measuring values. This allows monitoring of the functionality of the gas pressure sensor.

Preferably, the open end or the open ends of the fluid conduit system is/are closed prior to injection/extraction of the fluid test quantity, i. e. all liquid and gas openings towards the atmosphere are closed by closing a corresponding valve or by pinching them off with a clamping forceps. This prevents the fluid from escaping or entering through the open end of the fluid conduit during injection/extraction of the fluid test quantity.

The open end or the open ends of the fluid conduit can be closed manually by being pinched off, or automatically by means of controllable valves and opened again after completion of the monitoring process.

After injection the same fluid test quantity can be extracted again from the fluid conduit such that in a leakage-free fluid conduit the initial state is restored.

According to a preferred aspect of the method the fluid test quantity is injected or extracted at a defined timing. During injection/extraction of the fluid test quantity the time dependence of the fluid gas pressure is measured and recorded. Finally comparing is effected by comparison of the time dependence of the gas pressure or the gas differential pressure with a stored fluid-pressure standard value or fluid pressure-difference standard value. Injection/extraction of the fluid may be carried out at a constant flow rate but also at a varying flow rate. The fluid test quantity can be injected/extracted rapidly and pulsedly and/or at a progressively or degressively increasing or decreasing flow rate. By injection/extraction of the fluid test quantity at a defined timing and simultaneous recording of the time dependence of the gas pressure further information on the fluid conduit are collected. For example, from the pulse delay and pulse deformation of the pulsed fluid injection/extraction the elasticity of the overall fluid conduit system may be inferred, which, in turn, allows conclusions with respect to the length of the fluid conduit to be drawn. The gas permeability of the protector membrane can also be determined in this way.

The injecting and extracting of the fluid test quantity can be performed by a continuous volume modulation with a constant amplitude $\Delta V$ at a constant frequency. The total gas volume can then be determined using the first derivate of the term $PV = nRT$:

$$\frac{\delta P}{\delta V} = \frac{nRT}{V^2}$$

By superposing a modulated liquid volume "signal" of constant frequency it is possible to determine the total gas volume during a substantial liquid volume changement, i.e. during pumping a solution to a patient.

The fluid pressure can be determined by means of a liquid pressure sensor. Further parameters of the fluid volume can be measured and compared with standard values to obtain more detailed information on the fluid conduit and the fluid or the fluids contained therein. For this purpose e. g. both the liquid pressure and the gas pressure can be measured. Further, the liquid level can be determined, e. g. in a drip chamber. Moreover, the gas temperature can be determined which also substantially changes at relatively large injected fluid test quantities and should be taken into consideration with regard to temperature compensation.

When the gas temperature of the gas bubble can be determined, the gas volume is calculated as follows in consideration of the gas temperature:

$$V_0 = \Delta V\, P_2\, T_0 \,/\, (P_1\, \Delta T - T_0\, (P_2 - P_1)), \qquad (2)$$

where

$T_0$   is the temperature of the gas bubble prior to injection/extraction of the fluid test quantity, and

$\Delta T$   is the temperature change of the gas bubble after injection/extraction of the fluid test quantity.

According to independent method claim 6, monitoring of a fluid conduit system connected to a medical instrument can, alternatively to the method described above, be realized with the following steps: continuous measuring of the fluid pressure in the fluid conduit system during the entire process. During the measurement a fluid is injected into or extracted out of the fluid conduit until a predetermined fluid pressure is reached. The fluid quantity injected into/extracted out of the fluid conduit system until the predetermined fluid pressure is reached is compared with a specified fluid-quantity standard value. Finally, the deviation of the injected/extracted fluid quantity from the stored fluid-quantity standard value is issued. When this method is applied, a predetermined known fluid pressure or a fluid pressure difference is thus specified, whereas the volume change required for reaching this fluid pressure is measured. Tests have shown that this method, which incorporates the gas temperature that is to be simultaneously measured, is very exact such that even small deviations from aimed values can be reliably determined.

According to a further independent apparatus claim a medical instrument comprises a monitoring apparatus for monitoring a fluid conduit system. The medical instrument further comprises a fluid pump connectable to the fluid conduit and a pressure sensor connectable to the fluid conduit. The monitoring apparatus is connected with the fluid pump and can induce the fluid pump to inject or extract a fluid test quantity into or out of the fluid conduit. The monitoring apparatus is further connected with the pressure sensor and comprises a signal evaluation unit which compares, as a function of the fluid test quantity injected into or extracted out of the fluid conduit, the pressure change measured by the pressure sensor with a stored standard value and issues the comparison result. Most medical instruments adapted to supply patients with liquids are, as standard, already provided with a fluid pump configured for this purpose. A pressure sensor for connection to the fluid conduit of a fluid conduit kit is also normally provided as standard. Only a monitoring apparatus must be arranged in the medical instrument, said monitoring apparatus being connected with the fluid pump and the pressure sensor. The monitoring apparatus is programmed such that it carries out the monitoring process described above. In this way, a monitoring apparatus for monitoring the correct selection or tightness and other parameters of the fluid conduit system can be realized in a medical instrument at a relativey low extra expenditure.

The common concept of all independent claims is the injection/extraction of a known fluid test quantity to get an information about the status of the fluid conduit system, e.g. the gas volume, the liquid volume, the total conduit volume, the conduit tightness, the elasticity and other parameters.

Preferably, the pressure sensor is a gas pressure sensor which measures the gas pressure in a gas bubble at a location of the fluid conduit system. In addition, further sensors may be provided, preferably a gas temperature sensor, a liquid level sensor and/or a liquid pressure sensor. The more sensors of different types are arranged on the fluid conduits, the more information is available for evaluation of all sensor signals concerning the structure of the fluid conduit system and the ratios in the fluids.

According to a preferred aspect, the monitoring apparatus is, for this purpose, connected with all sensors. The monitoring apparatus compares, as a function of the fluid test quantity ejected/extracted by the fluid pump into or out of the fluid conduit system, all sensor-measured values with stored sensor standard values. The result of these comparisons is finally issued and evaluated.

To collect further information on the fluid conduit system and the fluid in the fluid conduits, the time dependence of the sensor signals are, according to a preferred aspect, compared with stored signal standard time dependence in the signal evaluation unit, and corresponding comparison signals are issued.

By evaluation of the time dependence of the sensor signals at known timing of the injection/extraction of the fluid test quantity, additional information on the fluid conduits or the fluid in the fluid conduits is collected. Owing to these additional information, the accuracy und redundancy of the measurement are improved and additional parameters of the fluid conduit system and/or its operating state can be determined.

Hereunder several embodiments of the invention are explained in detail with reference to the drawings in which:

Fig. 1 shows a schematic diagram of a first embodiment of a medical instrument according to the invention,

Fig. 2 shows a second embodiment of a medical instrument comprising a simple monitoring apparatus,

Fig. 3 shows a third embodiment of a medical instrument comprising a monitoring apparatus having a gas pressure sensor,

Fig. 4 shows a flowchart of a simple method for monitoring an extracorporeal fluid conduit, and

Fig. 5 shows a flowchart of a second embodiment of a method for monitoring an extracorporeal fluid conduit connected to a medical instrument.

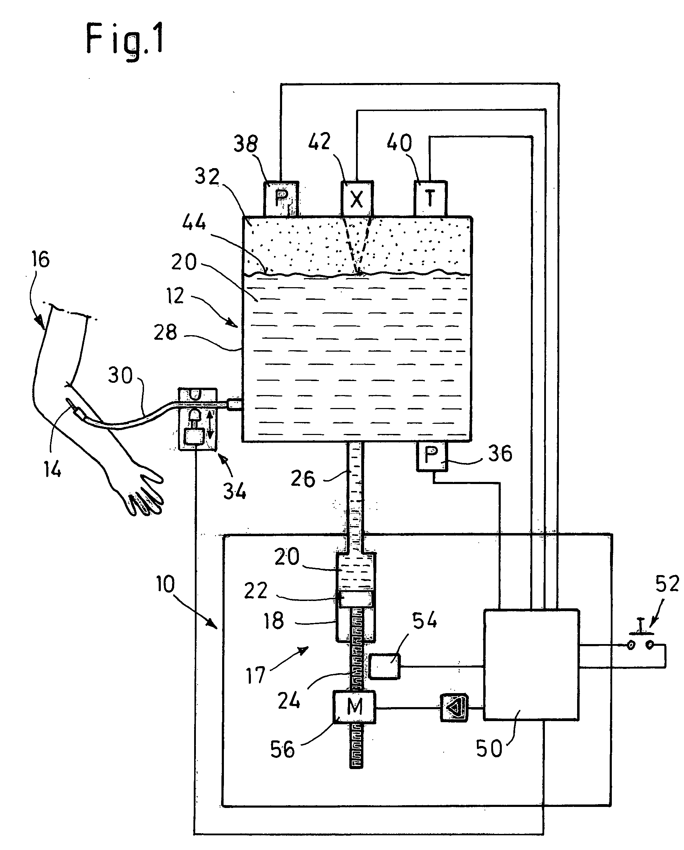

Fig. 1 shows a schematic diagram of a medical instrument 10 comprising an extracorporeal fluid conduit system 12 at the open end 14 of which a needle is arranged which provides access to a blood vessel of a patient 16. In the present case, the medical instrument 10 serves for dosed supply of a liquid fluid 20, e. g. a concentrated medicine, to the patient's body 16. The medical instrument may also serve for pumping off body liquid or for treatment of a body liquid, i. e. for both pumping it off the body and return it to the body after the treatment of the body liquid. Examples of such instruments are infusion instruments, dialysis instruments etc. The fluid conduit to be monitored can also be part of a degassing system of a dialysis apparatus.

The overall fluid conduit system 12 is used only once to prevent infections and is then disposed of. For each application one or even a plurality of different fluid conduit kits or systems are available which normally comprise all parts getting in contact with a fluid, namely hoses, containers, needles, syringe bodies, filter parts etc. In the present embodiment shown in Fig. 1, the fluid conduit system 12 comprises a syringe pump 17 with a syringe 18 including a piston 22 and a piston rod 24 as well as all fluid-carrying conduits 26,28,30 between the medical instrument 10 and the patient's body 16. The conduit 28 shown as a container in Fig. 1 serves as model. The fluids contained in conduit 28 are always a liquid 20 and a gas bubble 32 located above the liquid.

After installation of a new fluid conduit system 12 sensors and an electrical hose clamp 34 are attached to the corresponding fluid conduits 26-30. The sensors attached to a fluid conduit 28 are a liquid pressure sensor 36, a gas pressure sensor 38, a gas temperature sensor 40 and a liquid level sensor 42. The entire fluid conduit system 12 normally comprises only a single opening, namely in the form of a needle opening at the open end 14 of the conduit, but must be gastight and liquid-tight at all other locations. By means of the liquid level sensor 42 the level of the barrier layer 44 of liquid 20 and gas 32 is measured in the fluid conduit 28.

The medical instrument 10 comprises a monitoring apparatus 50 for controlling and monitoring purposes, the monitoring apparatus 50 being normally configured as a microprocessor. Further, a start switch 52, an encoder 54 as well as a drive motor 56 are provided. By means of the drive motor 56 the piston rod 24 of the syringe 18 is reciprocated. With the aid of the encoder 54 the absolute position of the piston rod 24 and thus the piston 22 is determined. Driving of the piston rod 24 can thus be monitored and checked. Using the start switch 52 testing of the fluid conduit system 12 is started.

By means of the electrical conduit clamp 34 the fluid conduit 30 can be completely squeezed together at a predetermined location and thus the flow of the liquid 20 and/or the gas 32 can be completely blocked.

The sensors 36-42, the encoder 54 and the start switch 52 are directly connected with the monitoring apparatus 50 via corresponding sensor lines. The motor 56 as well as the electrical conduit clamp 34 are connected via a respective control line with the monitoring apparatus 50 and can be directly controlled by the latter.

After installation of a new fluid conduit system 12 and attachment of all sensors 36-42 and the conduit clamp 34 to the corresponding fluid conduits 26-30 monitoring of the extracorporeal fluid conduit system 12 is initiated automatically or by pressing the start switch 52. First, the electrical conduit clamp 34 is set into the closing position by a corresponding control signal from the monitoring apparatus 50 such that the single open end of the fluid conduits 26-30 are closed. First, the initial values of all sensors 36-42 are read in and stored. Then, by transmission of the corresponding control signals from the monitoring apparatus 50 to the drive motor 56, a small quantity of fluid 20 from the syringe 18 is injected into the fluid conduit system 12. This process is monitored by evaluation of the encoder signals.

During and after injection of the fluid test quantity into the fluid conduit system 12 the sensor signals of all sensors 36-42 are collected in the monitoring apparatus, and the difference to the respective standard values is stored as differential values. In the monitoring apparatus 50 standard values of the liquid differential pressure, the gas differential pressure, the gas temperature and the liquid level are stored for the fluid conduit system 12. Further, a corresponding standard time dependence of the aforementioned parameters for the pulsed injection of a fluid test quantity at a certain timing is stored. The stored standard values and standard time dependences are compared with the time dependence of the aforementioned parameters in a signal evaluation unit of the monitoring apparatus 50. Depending on the degree of deviation of a measured parameter from its standard value or the standard time dependence the monitoring apparatus 50 issues an alarm signal. The alarm signal can be issued electronically and/or optically such that further operation of the extracorporeal fluid conduit system 12 is prevented and the operating personnel is warned.

Evaluation of the sensor signals can be completely or partly carried out by calculation, e. g. by application of equation (1) or (2). The thus determined values of the gas volume $V_0$ can then be displayed and/or compared with a stored gas-volume standard value, and the comparison result can be displayed and/or further processed.

After issue of the fluid pulse the syringe pump 17 can extract, i.e. suck in, the same fluid quantity such that the initial state is restored. The sensor signals determined during the extract process can also be correspondingly evaluated by the monitoring apparatus. The injection and extraction can be performed periodically and with a frequency lower than 10 Hz.

By interpretation of the type and degree of deviations conclusions can be drawn with respect to the type of disturbance. In this way not only a leak in the fluid conduit system 12 can be determined and localized, but further the extent of the gas volume 32 forming a gas bubble, the overall volume of the fluid conduit system 12 etc. can be calculated. In this way it is checked whether the correct fluid conduit kit or system has been used, whether the gas volume-to-liquid volume ratio is correct or whether e. g. too much gas has accummulated in a fluid conduit 26-30. Further, the functionality of the sensors 36-40 can be checked in this way. Altogether, the measuring method described offers the possibility to realize a monitoring method for extracorporeal fluid conduit systems of medical instruments, which monitoring method presents a very high degree of redundancy.

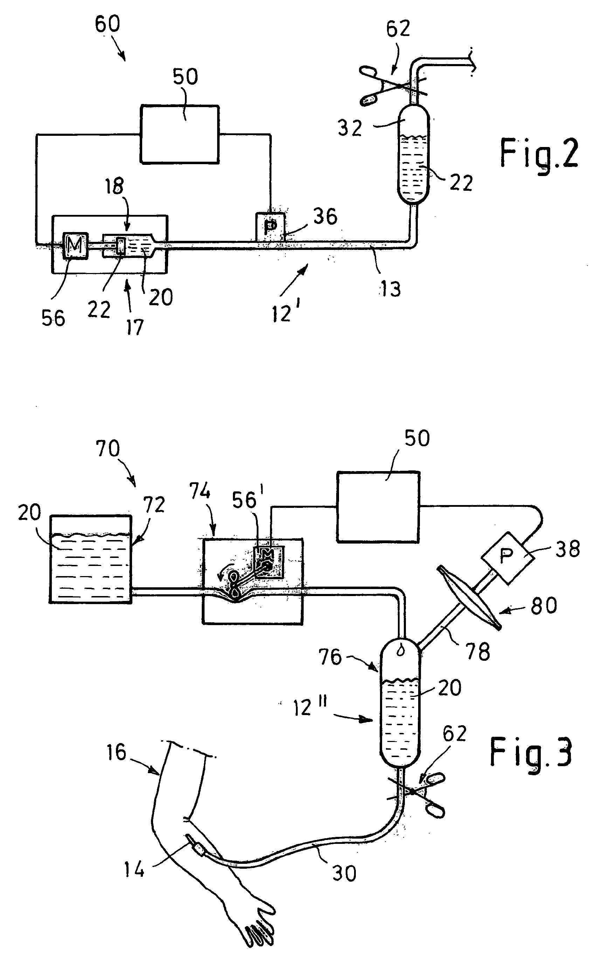

Fig. 2 shows a schematic diagram of the simplest embodiment of a medical instrument 16 comprising a fluid conduit kit 12'. The monitoring apparatus 50 controls the syringe pump 17. On the fluid conduit 13 a removable liquid pressure sensor 36 is arranged which measures the static liquid pressure. The fluid conduit 13 extending to a patient's body is closed near the open end by a clamping forceps 62. After closing of the open end of the fluid conduit 13 first the initial pressure $P_1$ is determined and stored. Then, the syringe pump 17 is controlled by the monitoring apparatus 50 such that a liquid pulse is transmitted into the fluid conduit kit 12'. The pressure curve is sensed by the liquid pressure sensor 36, the difference to the initial pressure $P_1$ is determined and compared in the monitoring apparatus 50 with a standard value or a standard curve of the liquid differential pressure. From the value and the curve of the measured liquid pressure and its deviation from the stored standard curve conclusions can be drawn with regard to the volume of gas 32 and liquid 20 as well as leakages.

Fig. 3 shows a third embodiment of a medical infusion instrument 70 frequently used in practice. The liquid 20 is pumped from an infusion solution container 72 through a peristaltic pump 74 to a drip chamber 76. The free end of the liquid conduit kit 12" is again closed by means of a clamping forceps 62. From the upper end of the drip chamber 76 a sensor line 78 branches off at the end of which a gas pressure sensor 38 is arranged. Both the sensor line 78 and the protector 80 are associated with the exchangeable fluid conduit kit 12". In the sensor line 78 a hydrophobic membrane is arranged as a gas sensor protector 80 which is gas-permeable in dry condition. In wet condition the membrane acts as a seal which is liquid-tight and gastight. The protector membrane protects the gas pressure sensor 38 against contamination by liquid. If the liquid level rises in an undesired manner into the sensor line 78 up to the protective membrane 80, the protector membrane 80 becomes moist, which makes it gastight and liquid-tight. In this condition the gas pressure sensor 38 senses measured values and transmits them to the monitoring apparatus 50, but these pressure values do no longer reflect the pressure in the liquid but strongly deviate from this pressure. While in conventional infusion instruments this condition cannot be reliably detected, the instrument 70 can regularly and reliably monitor the gas volume such that an imminent wetting of the protector membrane 80 by the liquid 20 is detectable in due time. By means of the monitoring method described above it can be reliably detected that the protector membrane has become gastight and liquid-tight by wetting with liquid such that further carrying-out of the treatment on the basis of incorrect pressure values is reliably prevented.

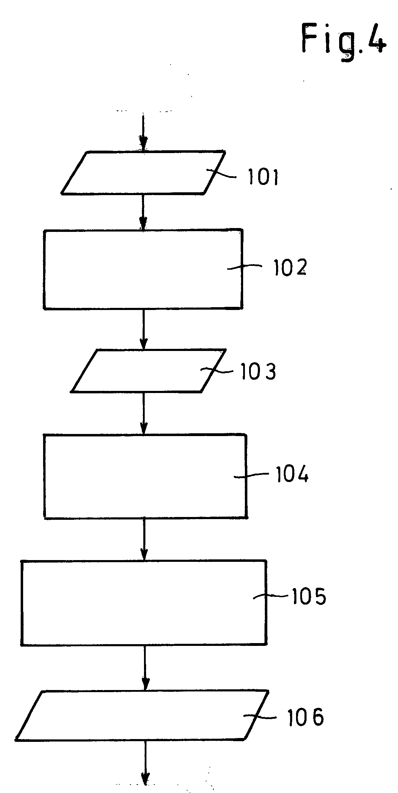

Fig. 4 shows the flowchart of the simplest form of a monitoring process. In a first process step 101 the initial pressure $P_1$ is read in by the monitoring apparatus. In the next step 102 the fluid pump injects a fluid test quantity $\Delta V$. Then the monitoring apparatus reads out the new pressure value $P_2$ from the pressure sensor. In the fourth process step 104 the fluid pump is returned to its initial position, i. e. the fluid test quantity $\Delta V$ is extracted from the fluid conduit system. In a fifth process step 105 the gas volume $V_0$ is determined from the parameters $\Delta V$ and $\Delta P = P_2 - P_1$. This volume can be determined by arithmetical operations according to equation (1) or by determination of the volume from performance maps, libraries and the like. In the last process step 106 the calculated gas volume $V_0$ is issued and, if necessary, compared with a gas-volume standard value.

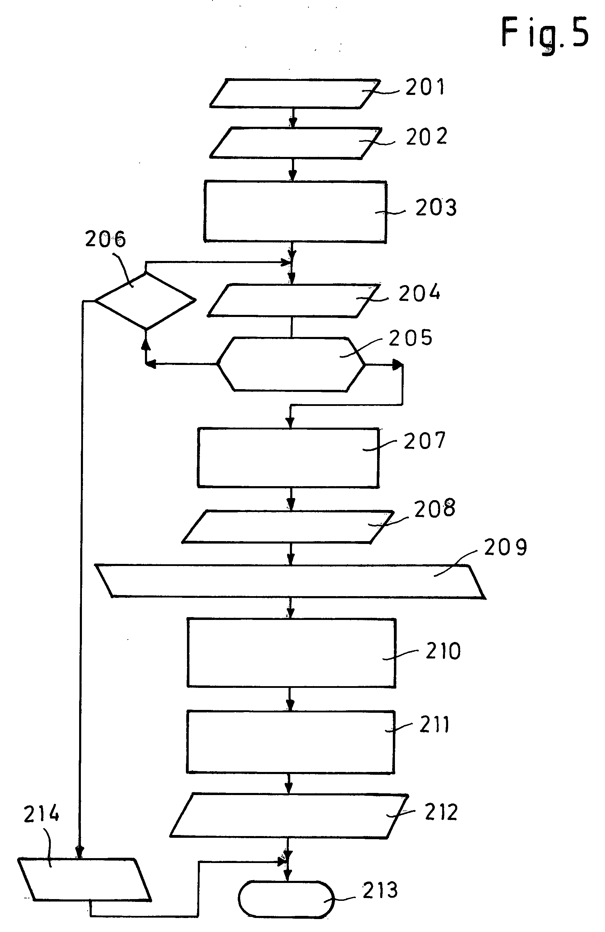

A second monitoring method, which is more extensive than the monitoring method shown in Fig. 4, is shown in Fig. 5. In a first process step 201 a value for the permissible deviation A is entered by the user or from a memory. In the next process step 202 the initial gas pressure $P_1$ and the initial gas temperature $T_0$ are measured. In the next process step 203 the pump supplies a liquid test quantity $\Delta V$. In the following process step 204 the pressure sensor reads the gas pressure P into the monitoring apparatus. In the next process step the difference between the initial gas pressure $P_1$ and the just measured gas pressure P is determined and compared with the accuracy coefficient A. If the pressure difference is $P-P_1 > A$, the following process step 207 is taken in which the liquid pump is stopped. If the pressure difference is $P-P_1 < A$, it is checked in a further process step 206 whether a specified time limit has been exceeded. If the specified time limit has not been exceeded, process step 204 is taken, i. e. a new measurement of the gas pressure is carried out. If the time limit has been exceeded, an alarm signal is issued in another process step 214. Process step 207 is followed by process step 208 in which a (last) measurement of the final gas pressure $P_2$ and the final gas temperature $T_1$ is carried out. In the following process step 209 the injected liquid test quantity $\Delta V$ is determined, e. g. read out from an encoder of the injection instrument. In the following process step 210 the injected liquid test quantity is sucked in again by the pump. In the following process step finally the gas volume $V_0$ is determined according to equation (2) from the initial gas pressure $P_1$, the final gas pressure $P_2$, the liquid test quantity $\Delta V$ and the gas temperatures $T_0$ and $T_1$. Finally, in a following process step 212 the determined gas volume value $V_0$ is issued, whereupon the monitoring process is stopped in its last process step 213, or will be repeated automatically and continuously.

By injecting and/or extracting a test volume of a fluid into the fluid conduit system, which should be closed, if necessary and possible, and simultaneously determining the pressure and other parameters, a large amount of information on the type, status and condition of the fluid conduit system and the fluids contained therein can be collected.

**Claims**

1. Method for monitoring a fluid conduit system (12) of a medical instrument (10), the method comprising the following steps:

measuring the initial fluid pressure ($P_1$) in the fluid conduit system (12),

injecting or extracting a fluid test quantity ($\Delta V$) into or out of the fluid conduit system (12),

measuring the final fluid pressure ($P_2$) in the fluid conduit system (12),

determining the pressure difference ($P_2-P_1$) between initial and final end pressures,

comparing the determined pressure difference ($P_2-P_1$) with a stored pressure-difference standard value, and

issueing the deviation of the determined pressure difference ($P_2-P_1$) from the stored pressure-difference standard value.

2. Method for monitoring a fluid conduit system (12) of a medical instrument (10), the fluid conduit system (12) comprising a liquid and a gas, the method comprising the following steps:

measuring the initial fluid pressure ($P_1$) in the fluid conduit system (12),

injecting or extracting a liquid fluid test quantity ($\Delta V$) into or out of the fluid conduit system (12),

measuring the final fluid pressure ($P_2$) in the fluid conduit system (12), and

determining the gas volume ($V_0$) by

$$V_0 = -\Delta V\, P_2 / (P_2 - P_1).$$

3. Method according to claim 1 or 2, **characterized in that**, prior to the process step of injection or extraction, closing all open ends (14) of the fluid conduit system (12).

4. Method according to one of claims 1-3, **characterized in that** injection or extraction is effected by pulsed supply of the fluid test quantity.

5. Method according to one of claims 1-4, **characterized by**

injecting or extracting of the fluid test quantity ($\Delta V$) at a defined timing,

measuring by recording the time dependence of the fluid pressure during injection or extraction and after injection or extraction of the fluid, and

comparing by comparison the time dependence of the gas pressure difference with a stored gas pressure-difference standard time dependence.

6. Method for monitoring a fluid conduit system (12) connected to a medical instrument (10), the method comprising the following steps:

measuring continuously the fluid pressure in the fluid conduit (12),

injecting or extracting a fluid into the fluid conduit system until a specified fluid pressure ($P_2$) has been reached,

comparing the fluid quantity ($\Delta V$) injected or extracted into or out of the fluid conduit system (12) until the specified fluid pressure with a specified fluid-quantity standard value is reached, and

issueing the deviation of the injected or extracted fluid quantity ($\Delta V$) from a stored fluid-quantity standard value.

7. Medical instrument comprising a monitoring apparatus (50) for monitoring a fluid conduit system (12), a fluid pump (17) connectable to the fluid conduit system (12), and a pressure sensor (38) connectable to the fluid conduit system (12), wherein

the monitoring apparatus (50) is connected with the fluid pump (17) and can induce the fluid pump (17) to inject or extract a fluid test quantity ($\Delta V$) into or out of the fluid conduit system (12), and

the monitoring apparatus (50) is connected with the pressure sensor (38) and comprises a signal evaluation unit which determines the pressure difference ($P_2$-$P_1$) caused by the fluid test quantity and measured by the pressure sensor (38) and compares the pressure difference ($P_2$-$P_1$) with a stored pressure-difference standard value and issues the comparison result.

8. Medical instrument according to claim 7, **characterized in that** the pressure sensor (38) is a gas pressure sensor which senses the fluid pressure in the fluid conduit system (12) and the monitoring apparatus (50) comprises a volume determination unit which determines the total volume ($V_0$) of the gas.

9. Medical instrument according to one of claims 7 or 8, **characterized in that** a gas temperature sensor (40), a liquid level sensor (42) and/or a liquid pressure sensor (36) are provided for connection to the fluid conduit system (12).

10. Medical instrument according to one of claims 7-9, **characterized in that** the monitoring apparatus (50) is connected with all sensors (36-42) and compares, as a function of the fluid test quantity ($\Delta V$) injected or extracted by the fluid pump (17) into or out of the fluid conduit system (12), the sensor-measured values with the stored standard values and issues the comparison result.

11. Medical instrument according to one of claims 7-10, **characterized in that** the signal evaluation unit compares the time dependence of the sensor signals with stored signal standard time dependences and issues the comparison results.

12. Medical instrument according to one of claims 7-10, **characterized in that** the fluid conduit system (12) is an exchangeable extracorporeal conduit kit.

# Fig.1

Fig.2

Fig.3

Fig.4

# Fig.5

**European Patent**
**Office**

## PARTIAL EUROPEAN SEARCH REPORT

**Application Number**

which under Rule 45 of the European Patent Convention EP 01 12 9940
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 87 05225 A (KAMEN DEAN L)<br>11 September 1987 (1987-09-11)<br>* page 2, line 32 - page 4, line 10 *<br>* page 5, line 9 - line 17 *<br>* page 8, line 25 - page 9, line 4 *<br>* page 9, line 16 - line 25; figures 1,8 *<br>* page 10, line 22 - line 35; figure 5 *<br>* page 12, line 1 - line 30 * | 7-12 | A61M1/36<br>A61M5/168 |
| A | EP 0 330 761 A (FRESENIUS AG)<br>6 September 1989 (1989-09-06)<br>* column 3, line 50 - column 4, line 11; figures 1,2 * | 7 | |
| A | WO 96 25214 A (FELDSEIN THOMAS M ;WILKERSON DOUGLAS L (US); ADOLF WAYNE F (US); A) 22 August 1996 (1996-08-22)<br>* page 83, line 18 - page 85, line 2; figures 6,19 * | 7 | |

**TECHNICAL FIELDS SEARCHED (Int.Cl.7)**

A61M

### INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 15 May 2002 | Zeinstra, H |

EPO FORM 1503 03.82 (P04C07)

**European Patent Office**

**INCOMPLETE SEARCH
SHEET C**

Application Number

EP 01 12 9940

```
Claim(s) not searched:
        1-6

Reason for the limitation of the search (non-patentable invention(s)):

Article 52 (4) EPC - Method for treatment of the human or animal body by
surgery
Article 52 (4) EPC - Method for treatment of the human or animal body by
therapy
The instrument is implicitely connected to a patient, and therefore
induces a direct therapeutic effect on the patient.
When it is connected by means of a needle, it has also a surgical effect.
```

**ANNEX TO THE EUROPEAN SEARCH REPORT
ON EUROPEAN PATENT APPLICATION NO.**     EP 01 12 9940

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2002

| Patent document cited in search report | | Publication date | Patent family member(s) | | Publication date |
|---|---|---|---|---|---|
| WO 8705225 | A | 11-09-1987 | US | 4778451 A | 18-10-1988 |
| | | | AU | 7161887 A | 28-09-1987 |
| | | | AU | 7162187 A | 28-09-1987 |
| | | | AU | 7164687 A | 28-09-1987 |
| | | | CA | 1278843 A1 | 08-01-1991 |
| | | | CA | 1282737 A2 | 09-04-1991 |
| | | | DE | 3770221 D1 | 27-06-1991 |
| | | | DE | 3773091 D1 | 24-10-1991 |
| | | | DE | 3775318 D1 | 30-01-1992 |
| | | | EP | 0258424 A1 | 09-03-1988 |
| | | | EP | 0259464 A1 | 16-03-1988 |
| | | | EP | 0262182 A1 | 06-04-1988 |
| | | | JP | 63503116 T | 17-11-1988 |
| | | | JP | 63503117 T | 17-11-1988 |
| | | | JP | 2551803 B2 | 06-11-1996 |
| | | | JP | 1501446 T | 25-05-1989 |
| | | | US | 4816019 A | 28-03-1989 |
| | | | US | 4804360 A | 14-02-1989 |
| | | | US | 5401342 A | 28-03-1995 |
| | | | US | 4808161 A | 28-02-1989 |
| | | | WO | 8705223 A1 | 11-09-1987 |
| | | | WO | 8705224 A1 | 11-09-1987 |
| | | | WO | 8705225 A2 | 11-09-1987 |
| | | | US | 4826482 A | 02-05-1989 |
| | | | US | 5533389 A | 09-07-1996 |
| | | | US | 5526844 A | 18-06-1996 |
| | | | US | 5575310 A | 19-11-1996 |
| | | | US | 5193990 A | 16-03-1993 |
| | | | US | 5116021 A | 26-05-1992 |
| | | | US | 5178182 A | 12-01-1993 |
| | | | US | 5211201 A | 18-05-1993 |
| | | | US | 5349852 A | 27-09-1994 |
| | | | US | 5222946 A | 29-06-1993 |
| | | | US | 5353837 A | 11-10-1994 |
| | | | US | 5195986 A | 23-03-1993 |
| | | | US | 5241985 A | 07-09-1993 |
| | | | US | 5364371 A | 15-11-1994 |
| EP 0330761 | A | 06-09-1989 | DE | 3806248 A1 | 07-09-1989 |
| | | | DE | 3883965 D1 | 14-10-1993 |
| | | | EP | 0330761 A1 | 06-09-1989 |
| | | | ES | 2045077 T3 | 16-01-1994 |
| | | | JP | 2007938 A | 11-01-1990 |
| WO 9625214 | A | 22-08-1996 | US | 5591344 A | 07-01-1997 |
| | | | AU | 698545 B2 | 29-10-1998 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**
EP 01 12 9940

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2002

| Patent document cited in search report | Publication date | Patent family member(s) | | Publication date |
|---|---|---|---|---|
| WO 9625214 A | | AU | 4444996 A | 22-08-1996 |
| | | AU | 4977996 A | 04-09-1996 |
| | | CA | 2168629 A1 | 14-08-1996 |
| | | DE | 19605260 A1 | 28-11-1996 |
| | | GB | 2299026 A ,B | 25-09-1996 |
| | | GB | 2310613 A ,B | 03-09-1997 |
| | | GB | 2310614 A | 03-09-1997 |
| | | GB | 2310615 A ,B | 03-09-1997 |
| | | GB | 2310602 A ,B | 03-09-1997 |
| | | GB | 2310616 A ,B | 03-09-1997 |
| | | GB | 2310617 A ,B | 03-09-1997 |
| | | GB | 2310618 A ,B | 03-09-1997 |
| | | GB | 2320210 A ,B | 17-06-1998 |
| | | GB | 2320209 A ,B | 17-06-1998 |
| | | JP | 9000618 A | 07-01-1997 |
| | | JP | 2002503115 T | 29-01-2002 |
| | | WO | 9625214 A1 | 22-08-1996 |
| | | US | 6146523 A | 14-11-2000 |
| | | US | 6153102 A | 28-11-2000 |
| | | US | 5674404 A | 07-10-1997 |
| | | US | 5705066 A | 06-01-1998 |
| | | US | 5645734 A | 08-07-1997 |
| | | US | 5690831 A | 25-11-1997 |
| | | US | 5658456 A | 19-08-1997 |
| | | US | 5707086 A | 13-01-1998 |
| | | US | 5674397 A | 07-10-1997 |
| | | US | 5630935 A | 20-05-1997 |
| | | US | 5690821 A | 25-11-1997 |
| | | US | 5725776 A | 10-03-1998 |
| | | US | 5670050 A | 23-09-1997 |
| | | US | 5702606 A | 30-12-1997 |
| | | US | 5674390 A | 07-10-1997 |
| | | US | 5651893 A | 29-07-1997 |
| | | US | 5788099 A | 04-08-1998 |
| | | US | 5714060 A | 03-02-1998 |
| | | US | 5932110 A | 03-08-1999 |
| | | US | 5788851 A | 04-08-1998 |
| | | US | 5783072 A | 21-07-1998 |
| | | US | 5716531 A | 10-02-1998 |
| | | US | 5762782 A | 09-06-1998 |
| | | US | 5863421 A | 26-01-1999 |
| | | US | 5932103 A | 03-08-1999 |

EPO FORM P0459

For more details about this annex : see Official Journal of the European Patent Office, No. 12/82